Europäisches Patentamt

European Patent Office

Office européen des brevets

(19)

(11) Numéro de publication: **0 105 784**
**A1**

## (12) DEMANDE DE BREVET EUROPEEN

(21) Numéro de dépôt: 83401846.7

(22) Date de dépôt: 22.09.83

(51) Int. Cl.³: **A 61 N 1/36**
**A 61 N 1/08**

(30) Priorité: 23.09.82 FR 8216034

(43) Date de publication de la demande:
18.04.84 Bulletin 84/16

(84) Etats contractants désignés:
CH DE GB IT LI SE

(71) Demandeur: SOCIETE EUROPEENNE DE PROPULSION
Société Anonyme dite:
3, Avenue du Général de Gaulle
F-92800 Puteaux(FR)

(72) Inventeur: Delle-Vedove, Daniel
3, Chemin de la Pointe aux Anglais
F-78126 Aulnay Sur Mauldre(FR)

(72) Inventeur: Lallemand, Yves
34, rue de Bizy
F-27200 Vernon(FR)

(72) Inventeur: Hubert, Olivier
14, place Etienne Pernet
F-75015 Paris(FR)

(74) Mandataire: Joly, Jean Jacques et al,
CABINET BEAU DE LOMENIE 55, rue d'Amsterdam
F-75008 Paris(FR)

(54) Procédé et dispositif de détection de la réponse du coeur à une impulsion électrique de stimulation.

(57) On recueille, après la fin de l'impulsion de stimulation, un premier signal v qui représente le potentiel d'électrode en circuit ouvert et dont la variation dans le temps t, à partir d'un instant origine $t_o$ est de forme sensiblement logarithmique : $v = v_o + k.\text{Log } t/t_o$, $v_o$ étant la veleur de v à l'instant $t_o$ et k étant un paramètre lié notamment à la durée et à l'amplitude de l'impulsion de stimulation, on convertit (en 11-14) le premier signal v en un second signal dont la variation dans le temps est sensiblement linéaire, et on détecte dans ce second signal une composante non linéaire représentant le signal produit par la contraction provoquée du coeur.

./...

Fig.1

<u>Procédé et dispositif de détection de la réponse du coeur à une</u>
<u>impulsion électrique de stimulation.</u>

La présente invention concerne la détection de la réponse du coeur à une impulsion électrique de stimulation.

Le domaine d'application de l'invention est celui des stimulateurs cardiaques, en particulier les stimulateurs cardiaques implantables de tous types.

Un stimulateur cardiaque consiste en un générateur d'impulsions électriques de stimulation connecté à une électrode placée en contact avec les tissus excitables du muscle cardiaque. La contraction du coeur a lieu lorsque l'énergie électrique délivrée à l'électrode dépasse un certain seuil (seuil de stimulation) qui est susceptible de varier au cours du temps. La même électrode est généralement utilisée aussi pour détecter les contractions spontanées du coeur afin d'inhiber le fonctionnement du stimulateur lorsque le coeur bat spontanément au-dessus d'un certain rythme.

Il serait également souhaitable de détecter les contractions provoquées, c'est-à-dire celles produites en réponse à une impulsion de stimulation, afin, notamment, d'adapter l'énergie fournie par le stimulateur aux besoins réels du coeur et, par là, de réduire la consommation d'énergie électrique. Toutefois, cette détection est rendue très difficile du fait que le passage du courant de stimulation provoque une polarisation électrique de l'interface électrode-coeur, c'est-à-dire une accumulation d'ions à la surface de l'électrode équivalente à la charge d'un condensateur. La disparition de cette charge après le passage du courant de stimulation (dépolarisation) est plus ou moins rapide suivant l'intensité du courant inverse succédant au courant de stimulation mais sa durée est telle (par exemple de l'ordre de 200 ms) que la tension de dépolarisation possède, au moment de la contraction provoquée, une amplitude de plusieurs centaines de mV très supérieure à la variation de potentiel (quelques mV) produite par la contraction. De ce fait, la réponse du coeur à l'impulsion de stimulation est "masquée" par la dépolarisation de l'électrode.

Pour détecter cette réponse sans être gêné par la tension de dépolarisation, il a été proposé d'utiliser au moins une électrode de détection différente de l'électrode de stimulation. L'inconvénient évident de cette solution consiste dans la nécessité de la présence permanente de plusieurs électrodes.

Il a aussi été proposé d'appliquer l'impulsion de stimulation et de détecter la contraction provoquée, en utilisant une même électrode et en combattant la dépolarisation de cette électrode.

Ainsi, les documents FR 2 241 287 et EP 0057944 proposent d'appliquer à l'électrode un courant de signe opposé à celui du courant de stimulation. On parvient certes ainsi à réduire la durée de la dépolarisation pour que la tension de dépolarisation résiduelle, au moment de la contraction provoquée, soit suffisamment faible pour ne pas masquer la réponse du coeur. Toutefois, l'application d'un courant opposé à celui de stimulation peut donner lieu à un phénomène d'électrolyse avec passage d'ions métal dans le coeur et possible corrosion de l'électrode. Dans le document FR 2 374 023, il est aussi proposé d'accélérer la dépolarisation par passage d'un courant inverse, la tension d'électrode étant mesurée en vue de la détection lorsque la variation de tension, en fin de dépolarisation, est lente. Toutefois, ce dernier dispositif utilise des circuits de mesure qui nécessitent un réglage fonction de la nature de l'électrode utilisée.

La présente invention a pour but de permettre la détection de contraction provoquée, sans électrode supplémentaire et sans les inconvénients des dispositifs connus qui combattent la dépolarisation de l'électrode.

Ce but est atteint grâce à un procédé de détection selon lequel, conformément à l'invention,

- on recueille, après la fin de l'impulsion de stimulation, un premier signal v qui représente le potentiel d'électrode en circuit ouvert et dont la variation dans le temps t, à partir d'un instant origine $t_o$ est de forme sensiblement logarithmique : $v = v_o + k.Log \frac{t}{t_o}$, $v_o$ étant la valeur de v à l'instant $t_o$ et k étant un paramètre lié notamment à la durée et à l'amplitude de l'impulsion de stimulation,

- on convertit le premier signal v en un second signal dont la variation dans le temps est sensiblement linéaire et,

- on détecte dans ledit second signal une composante non linéaire représentant le signal produit par la contraction provoquée du coeur.

L'invention est basée sur la constatation que la tension de dépolarisation spontanée (c'est-à-dire en circuit ouvert) de l'électrode varie dans le temps de façon sensiblement logarithmique quelle que soit la nature de l'électrode utilisée. La linéarisation du signal représentant la tension d'électrode en circuit ouvert permet alors une détection facile du signal produit par la contraction provoquée du coeur.

Suivant un premier mode de réalisation de l'invention, cette linéarisation comprend une phase de transformation par une fonction f de type exponentiel : $f(x) = Ae^{Kx}$, A et K étant deux paramètres. Pour obtenir un second signal linéaire, il faut soit atténuer le signal v par un facteur $\alpha$ tel que $\alpha kK=1$ -.de sorte que $f(v)$ est proportionnel à $e^{\alpha kK \, Log \frac{t}{t_o}}$ , c'est-à-dire à t - soit ajuster la valeur de K afin que $kK = 1$.

Suivant un second mode de réalisation de l'invention, la linéarisation comprend une phase de génération d'un signal de référence $v_{ref}$ variant de façon logarithmique tel que $v_{ref} = v'_o + K' \, Log \frac{t}{t_o}$ , $v'_o$ étant la valeur de $v_{ref}$ à l'instant $t_o$ et K' étant un paramètre, et une phase de comparaison du premier signal v avec le signal de référence. Pour obtenir un second signal linéaire, il faut soit atténuer le signal v par un facteur $\alpha'$ tel que $\alpha'k = K'$, soit ajuster la valeur de K' de sorte que $K' = k$.

Pour la détection, dans le second signal, d'une composante linéaire représentant l'information recherchée, on procède notamment à un filtrage destiné à extraire cette composante et à une comparaison de la grandeur filtrée avec une valeur de seuil prédéterminée.

L'invention a aussi pour but de fournir un dispositif permettant la mise en oeuvre du procédé défini plus haut.

Ce but est atteint grâce à un dispositif qui comporte :

- un interrupteur commandé destiné à placer l'électrode en circuit ouvert en réponse à la réception d'un signal de commande , et

- un circuit de détection comprenant un circuit d'entrée relié à l'électrode pour recueillir un premier signal v qui représente le potentiel d'électrode; un circuit convertisseur recevant ledit premier signal et fournissant sur sa sortie un second signal variant dans le temps de façon sensiblement linéaire lorsqu'il reçoit un signal variant dans le temps de façon sensiblement logarithmique; et un détecteur recevant ledit second signal et destiné à détecter dans celui-ci une composante non linéaire représentant le signal produit par la contraction provoquée du coeur.

D'autres particularités et avantages du procédé et du dispositif selon l'invention ressortiront à la lecture de la description faite ci-après, à titre indicatif mais non limitatif, en référence aux dessins annexés sur lesquels :

- la figure 1 est un schéma d'un mode de réalisation d'un dispositif de détection conforme à l'invention;

- les figures 2 à 4 illustrent les formes d'onde de signaux en différents points du dispositif de la figure 1, et

- la figure 5 est un schéma d'un autre mode de réalisation d'un dispositif de détection conforme à l'invention.

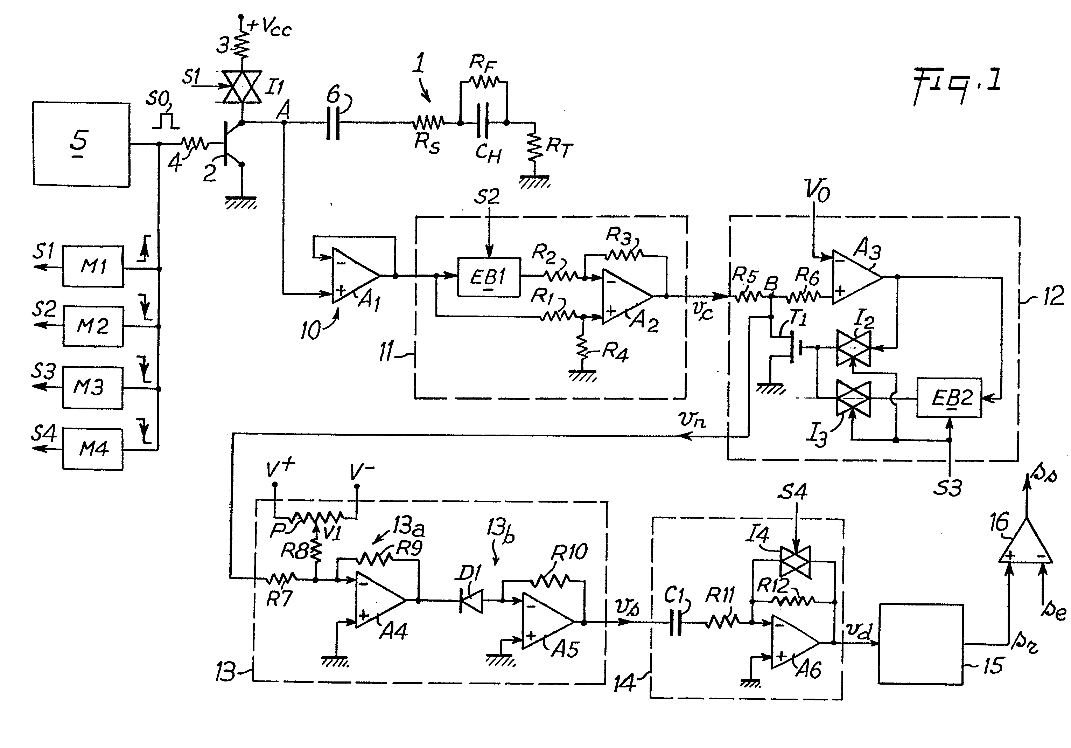

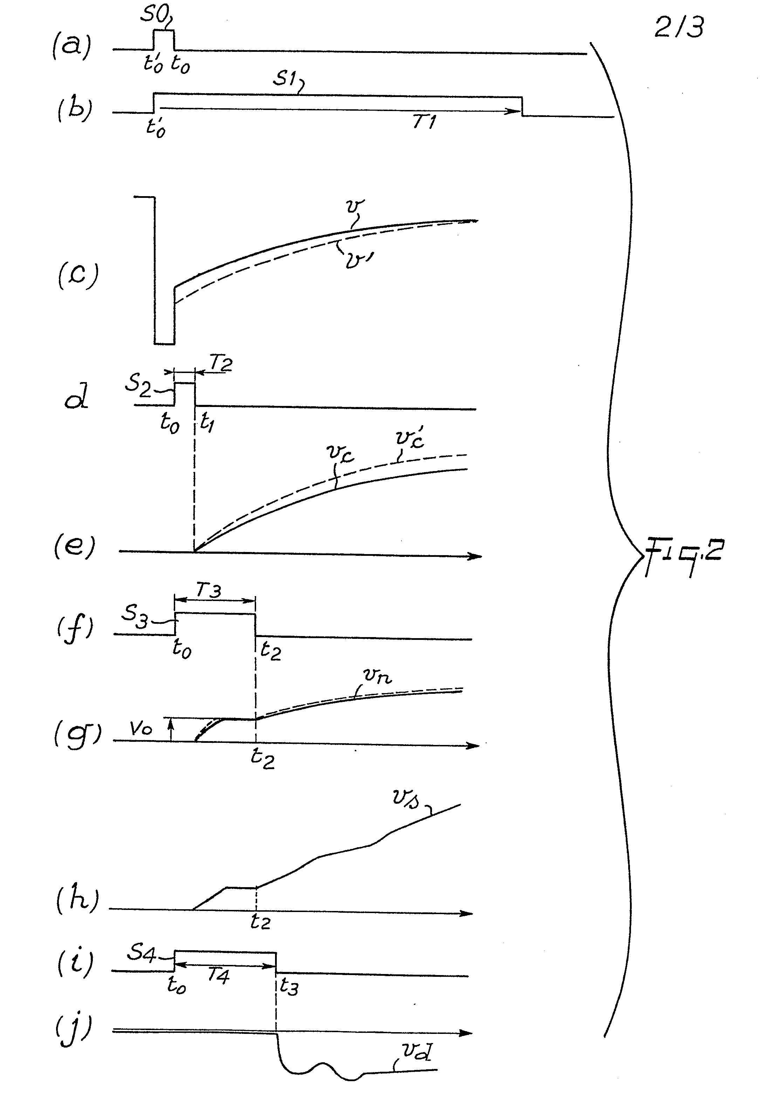

Sur la figure 1, on a représenté en 1 le schéma équivalent d'une électrode ou sonde de stimulation en contact avec le coeur d'un patient. La résistance série $R_S$ représente la résistance du fil de sonde. Le circuit parallèle formé par le condensateur $C_H$ et la résistance $R_F$ représente l'interface entre la sonde et le coeur et se trouve en série entre $R_S$ et la résistance $R_T$ des tissus du coeur. Un transistor 2 a son collecteur relié par une résistance 3 à une borne portée à un potentiel positif $V_{cc}$ et son émetteur relié à une borne portée à un potentiel de référence (masse). La base du transistor 2 est reliée par une résistance 4 à la sortie d'un circuit de commande 5. Un condensateur 6 est branché entre le collecteur du transistor 2 et l'électrode 1. Les éléments qui viennent d'être décrits sont les constituants usuels d'un stimulateur cardiaque classique. Lorsque le transistor 2 est bloqué, le le condensateur 6 se charge sous la tension $V_{cc}$. Une impulsion de stimulation SO produite par le circuit de commande (ligne a de la figure 2) débloque le transistor 2; le condensateur 6 se décharge partiellement dans le coeur, ce qui constitue l'impulsion négative de stimulation.

Conformation à l'invention, un interrupteur I1 est branché entre la borne au potentiel $V_{cc}$ et le point A commun au collecteur du transistor 2 et au condensateur 6. L'interrupteur I1 est un interrupteur de type analogique (par exemple un transistor) dont l'ouverture est commandée par un signal S1 (ligne b de la figure 2). Ce signal S1 est une impulsion produite à l'instant $t_o$ en réponse au front montant de SO par un multivibrateur monostable M1 relié à la sortie du circuit de commande 5. La durée T1 de S1 est choisie de manière à couvrir la production par le coeur du signal résultant de la contraction provoquée en réponse à SO. A titre indicatif, T1 est comprise entre 100 et 200 ms. Au bout du temps T1, l'interrupteur I1 se referme, ce qui permet la recharge du condensateur 6 à travers la résistance 3. Cette dernière est choisie suffisamment faible pour que le condensateur 6 soit rechargé avant la fin de la période réfractaire, c'est-à-dire le laps de temps suivant une impulsion (provoquée ou spontanée) pendant lequel le détecteur de signaux QRS spontanés est inhibé pour ne pas être perturbé par l'onde T. En effet, comme déjà indiqué, un stimulateur cardiaque classique est habituellement pourvu d'un détecteur de QRS spontanés qui bloque la génération d'impulsions électriques de stimulation lorsque le coeur bat spontanément au-dessus d'un certain rythme. La période réfractaire est généralement de l'ordre de 300 ms.

Le circuit de détection conforme à l'invention est branché au point A et comprend un circuit d'entrée 10, formé par un amplificateur A1 à grande impédance d'entrée, un circuit 11 de calage de zéro, un circuit 12 de mise à l'échelle, un amplificateur 13 de type "antilogarithmique", un dérivateur 14 et un filtre 15.

L'interrupteur I1 étant ouvert, le signal v prélevé au point A et recopié dans l'étage d'entrée 10 a la forme illustrée à la ligne c de la figure 2. Pendant la durée de SO, par exemple de l'ordre de 1 ms, (instant $t'_o$ à instant $t_o$), l'impulsion de

stimulation IS est appliquée au coeur. A l'instant $t_o$, le transistor 2 se bloque. Comme l'interrupteur I1 est ouvert, la tension v représente alors la tension d'électrode en circuit ouvert. A partir de l'instant $t_o$, la dépolarisation de l'électrode se traduit par une remontée de la tension v et la présente invention est fondée sur la constatation que la variation de v en fonction du temps t est de forme sensiblement logarithmique : $v = v_o + k \operatorname{Log} \frac{t}{t_o}$, $v_o$ étant la valeur de v à l'instant $t_o$ et k un coefficient. Il a été vérifié que la variation du potentiel de dépolarisation est de forme logarithmique quelle que soit la sonde utilisée et le type de stimulateur. Une linéarisation de v permet alors une détection plus aisée de la réponse du coeur. Toutefois, les valeurs de $v_o$ et de k varient suivant les types d'électrode et les conditions de fonctionnement, notamment suivant la durée et l'amplitude de l'impulsion de stimulation. Ainsi, avec un même stimulateur, la remontée de la tension d'électrode peut être de la forme $v' = v'_o + k' \operatorname{Log} \frac{t}{t_o}$, $v'_o$ étant différent de $v_o$ et k' étant différent de k (voir courbe en tirets à la ligne c de la figure 2). Aussi, avant la phase de linéarisation, on procède à la normalisation de v successivement par calage de zéro et mise à l'échelle.

Le calage de zéro consiste à imposer une valeur zéro à la tension v à un instant prédéterminé $t_1$ suivant l'instant $t_o$. A cet effet, le signal v provenant du circuit d'entrée A1 et reçu par le circuit 11 de calage de zéro est appliqué d'une part à un échantillonneur-bloqueur EB1 et, d'autre part, par l'intermédiaire d'une résistance R1, à l'entrée non inverseuse d'un amplificateur différentiel A2. L'entrée inverseuse de l'amplificateur A2 reçoit, par l'intermédiaire d'une résistance R2 le signal de sortie de EB1. La sortie de A1 est reliée à l'entrée inverseuse par une résistance R3 tandis que l'entrée non inverseuse de A1 est reliée à la masse par une résistance R4. L'échantillonneur-bloqueur EB1 est commandé par un signal S2 (ligne d de la figure 2) sous forme d'une impulsion produite en réponse au front descendant de SO par un multivibrateur M2 relié à la sortie du circuit de commande 5. La durée T2 de l'impulsion S2 est telle qu'à l'instant $t_1$ (front descendant de S2) EB1 est bloqué. A titre indicatif, on choisit $T_2 = 1$ ms. En sortie du circuit 11, on obtient donc une tension de la forme $v_c = k \, Log \frac{t}{t_o + T_2}$

ligne e de la figure 2). La courbe représentative de $v_c$ dépend de la valeur de k (voir la courbe en tirets $v'_c$ correspondant à $v'$).

La mise à l'échelle est réalisée au moyen du circuit atténuateur 12. Un diviseur de tension formé par une résistance R5 en série avec le trajet drain-source d'un transistor à effet de champ T1 est branché entre l'entrée du circuit 12 recevant $v_c$ et la masse. Le point commun B entre R5 et T1 forme la sortie du circuit 12 sur laquelle est délivré le signal $v_n$ mis à l'échelle (ligne g de la figure 2). Le point B est relié par une résistance R6 à l'entrée non inverseuse d'un amplificateur opérationnel A3 utilisé en comparateur et dont l'entrée inverseuse est reliée à une borne portée à un potentiel de référence prédéterminé réglable $V_o$. La sortie de A3 est reliée à l'électrode de commande (grille) du transistor T1 par l'intermédiaire, d'une part, d'un interrupteur commandé I2 et, d'autre part, du circuit série formé par un échantillonneur-bloqueur EB2 et un interrupteur commandé I3. Les interrupteurs I2 et I3 sont des interrupteurs de type statique commandés par un signal S3 (ligne f de la figure 2) sous forme d'une impulsion produite en réponse au front descendant de S0 par un multivibrateur monostable M3 relié à la sortie du circuit de commande 5. La durée T3 de l'impulsion S3 est supérieure à T2, par exemple de l'ordre de 5 ms. Les interrupteurs I2 et I3 sont commandés en synchronisme de sorte que l'un soit ouvert tandis que l'autre est fermé et réciproquement. Pendant la durée de S3 (instant $t_o$ à instant $t_2$), I2 est fermé et I3 est ouvert. A la fin de S3, I2 s'ouvre et I3 se ferme. L'impulsion S3 commande également l'échantillonneur-bloqueur EB2 pour bloquer le signal de sortie de celui-ci à la valeur acquise à l'instant $t_2$. Le fonctionnement de l'atténuateur est le suivant. Lorsque la tension $v_n$ atteint la valeur $V_o$, le comparateur A3 délivre un signal qui, appliqué à la grille de T1, commande la résistance drain-source de ce transistor de sorte que $v_n$ est maintenue à la valeur $V_o$. Au temps $t_2$, la tension de sortie de A3 est recopiée par l'échantillonneur-bloqueur EB2. I2 est ouvert, tandis que I3 est fermé pour maintenir la grille de T1 à la tension recopiée par EB2. La résistance drain-source de T1 reste ensuite constante. Par le circuit 12, on applique à la tension $v_c$ un coefficient d'atténuation $\alpha$ tel que, à l'instant $t_2$, la tension $v_n$ soit égale à $V_o$. On a alors $v_n = \alpha k \, \text{Log} \dfrac{t}{t_o + T_2}$ avec $\alpha = V_o / k \, \text{Log} \dfrac{t_2}{t_o + T_2}$ . A partir de l'instant $t_2$, la courbe de variation de $v_n$ est la même quelles que soient les valeurs de $v_o$ et k.

La tension normalisée $v_n$ est appliquée à l'entrée d'un circuit amplificateur antilogarithmique 13 c'est-à-dire un circuit à gain exponentiel. Le circuit 13 comprend un étage d'entrée 13a formant circuit de réglage de gain et un amplificateur antilogarithmique proprement dit 13b. L'étage d'entrée 13a comporte un amplificateur opérationnel A4 sur l'entrée inverseuse duquel est appliquée la somme de $v_n$ et d'une tension ajustable $V_1$. A cet effet, la sortie du circuit 12 est reliée à l'entrée inverseuse de A4 par une résistance R7 tandis que le curseur d'un potentiomètre P est relié à cette même entrée inverseuse par une résistance R8. Les extré-mités du potentiomètre P sont connectées à deux bornes portées à des potentiels fixes respectivement positif $V^+$ et négatif $V^-$. Une résistance R9 relie la sortie de A4 à son entrée inverseuse tandis que l'entrée non inverseuse de A4 est reliée à la masse. L'ampli-ficateur antilogarithmique 13b comprend, de façon connue en soi, un élément non linéaire formé par une diode D1 dont une borne est connectée à une première entrée (l'entrée inverseuse) d'un ampli-ficateur opérationnel A5 dont l'autre entrée est à la masse. Une résistance R10 relie la sortie de A5 à son entrée inverseuse. L'autre borne de D1 est reliée à la sortie de A4. Le circuit 13b produit sur sa sortie une tension : $v_s = a.e^{k \, ve}$, a et k étant des constantes et ve étant la tension appliquée à l'entrée de 13b. On a donc à partir du temps $t_2$, $v_s = a.e^{k(V_1 + v_n)}$ soit $v_s = ae^{kV_1} e^{\alpha kK \, \text{Log} \, t_2/t_o + T_2}$. Ainsi, si $\alpha kK = 1$, $v_s$ est, au-delà de $t_2$, une fonction sensiblement linéaire du temps t : $v_s = K_1 t$ (ligne h de la figure 2). L'égalité $\alpha kK = 1$ est obtenue en ajustant la valeur $V_o$, tandis que la pente $K_1$ de $v_s$ est réglable en ajustant le gain du circuit 13 par réglage de la valeur $V_1$ au moyen du potentiomètre P. La réponse du coeur (onde QRS) à l'impulsion de stimulation est alors traduite par une non-linéarité de $v_s$. Alors que cette réponse n'est pas discernable dans la courbe représentative de v, elle est au contraire parfaite-ment visible après la phase de linéarisation.

Afin d'accentuer le signal représentant la réponse du coeur, la tension $v_s$ est dérivée au moyen du dérivateur 14 branché à la sortie de l'amplificateur 13b. Ce dérivateur comprend un circuit série formé par un condensateur Cl et une résistance R11 et branché entre la sortie de A5 et l'entrée inverseuse d'un amplificateur opérationnel A6. Celui-ci a son entrée non inverseuse reliée à la masse tandis que sa sortie est reliée par une résistance R12 à l'entrée inverseuse. Un interrupteur I4 est branché en parallèle sur la résistance R12. Cet interrupteur est de type statique commandé par une impulsion S4 (ligne i de la figure 2) produite en réponse au front descendant de SO par un multivibrateur monostable M4 relié à la sortie du circuit de commande 5. L'impulsion S4 a une durée T4 supérieure à la durée T3 de l'impulsion S3. L'interrupteur I4 est maintenu fermé pendant la durée de S4, c'est-à-dire jusqu'à un instant $t_3$ retardé par rapport à $t_2$, afin d'éviter une saturation du dérivateur au moment $t_2$ du changement de pente de la tension de sortie de l'amplificateur antilogarithmique. Le signal $v_d$ en sortie du dérivateur 14 (ligne j de la figure 2) se présente donc, à partir de l'instant $t_4$ sous forme d'une tension continue à laquelle se superpose un signal non continu $s_r$ représentant la réponse du coeur.

Le filtre 15 est un filtre passe haut permettant d'extraire le signal $s_r$ de la composante continue de $v_d$. En sortie du filtre 15, le signal $s_r$ est aisément identifiable. Pour le détecter, il suffit par exemple d'appliquer le signal de sortie du filtre 15 à une entrée d'un comparateur 16 qui reçoit sur son autre entrée un signal de référence de seuil réglable $s_e$. Le comparateur 16 fournit un signal de sortie $s_s$ lorsque la valeur instantanée de $s_r$ dépasse la valeur de seuil $s_e$, traduisant ainsi la présence d'un signal de réponse du coeur à une contraction provoquée.

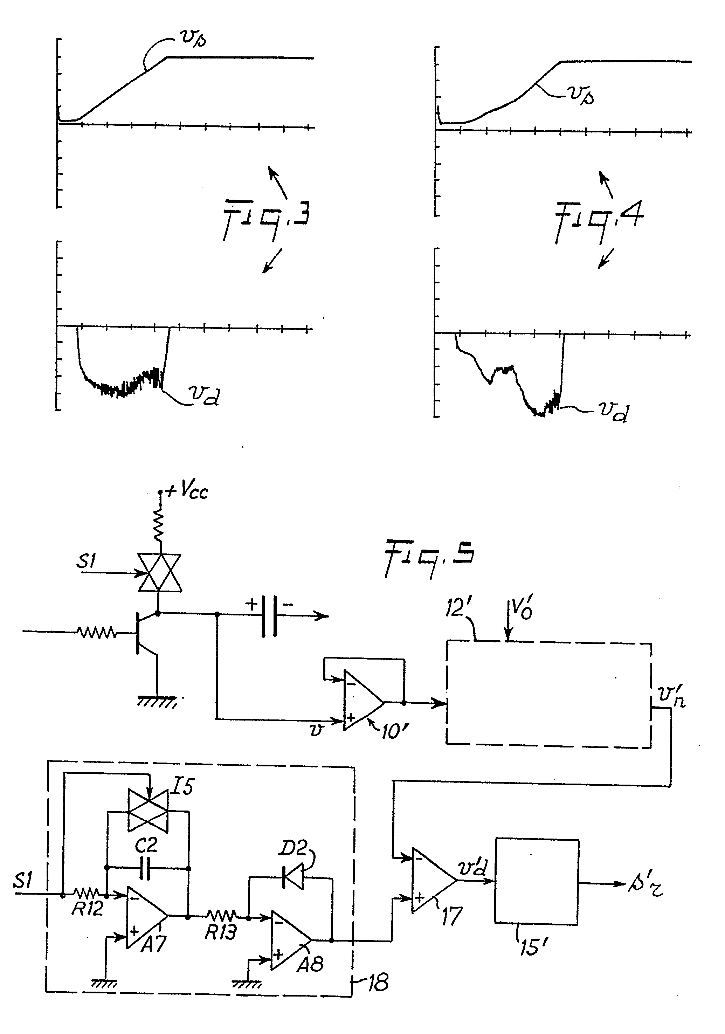

Des essais ont été effectués au cours d'une opération d'implantation d'un stimulateur sur un patient. La figure 3 illustre les signaux $v_s$, $v_d$ respectivement en sortie de l'amplificateur antilogarithmique, et en sortie du circuit dérivateur dans le cas d'absence de réponse du coeur, tandis que la figure 4 illustre ces mêmes signaux dans le cas de présence de réponse du coeur. Malgré les conditions défavorables d'enregistrement en salle d'opération, les figures 3 et 4 démontrent que la détection de la réponse du coeur est rendue possible sans aucune difficulté ou ambiguité grâce à la présente invention.

La détection d'un tel signal de réponse (QRS électrostimulé) peut être utilisée pour ajuster, comme connu en soi, l'énergie fournie par chaque impulsion de stimulation. Par exemple, cette énergie est diminuée d'un incrément lorsque le QRS électrostimulé est détecté, jusqu'à l'absence de détection, l'énergie étant alors augmentée d'un coup de plusieurs incréments. On peut également établir des statistiques relatives aux nombres de QRS spontanés et de QRS électrostimulés.

Une variante de réalisation d'un dispositif de détection conforme à l'invention est représentée très schématiquement sur la figure 5.

La tension v recopiée par le circuit d'entrée 10' analogue au circuit 10 est appliquée à l'entrée d'un circuit de mise à l'échelle 12' analogue au circuit 12. En sortie du circuit 12', on dispose d'une tension $v'_n$ qui varie de façon logarithmique

$$v'_n = v_o + \alpha k \, Log \frac{t}{t_o} .$$

Un amplifificateur opérationnel 17 , formant soustracteur, reçoit la tension $v'_n$ sur une première entrée (entrée inverseuse) et reçoit sur sa seconde entrée une tension w fournie par un générateur de fonction logarithmique 18 . La tension w varie de façon logarithmique en fonction du temps : $w = w_o + K' Log \frac{t}{t_o}$ , $w_o$ étant la valeur de w à l'instant $t_o$. Le fonctionnement du générateur 18  est par exemple commandé par l'impulsion S1. En sortie du comparateur 17 , on dispose donc d'une tension $v'_d = w_o - v_o + (K' - \alpha k) \, Log \frac{t}{t_o}$ . La valeur de $\alpha$ est ajustée par réglage de la tension de référence $v'_o$ appliquée au circuit 12' de manière que $K' = \alpha k$. On obtient ainsi un niveau continu $v'_d$ analogue à $v_d$, ce niveau continu étant filtré par un filtre 15' pour extraire la composante non continue $s'_r$.

Le générateur 18 comporte un étage 18a engendrant une tension en dent de scie et un étage 18b transformant cette tension en dent de scie en un signal variant suivant une fonction logarithmique. L'étage 18a comprend un amplificateur A7 qui reçoit le signal S1 sur son entrée inverseuse par l'intermédiaire d'une résistance R12 et dont l'entrée non inverseuse est reliée à la masse. Un condensateur C2 est branché entre la sortie de l'amplificateur A7 et son entrée inverseuse, en parallèle avec un interrrupteur statique I5 à ouverture commandée par l'impulsion S1. L'étage 18b est un circuit d'amplificateur logarithmique classique avec un amplificateur A8 dont l'entrée inverseuse reçoit le signal de sortie de l'étage 18a par l'intermédiaire d'une résistance R13, dont l'entrée non-inverseuse est reliée à la masse et dont la sortie est reliée à l'entrée inverseuse par une diode D2. Dès que l'interrupteur I5 est ouvert, la tension en sortie de l'étage 18a décroît de façon sensiblement linéaire pour revenir brusquement à son niveau initial au moment de la fermeture de I5 (fin de S1). Pendant la durée de S1, le signal variant de façon linéaire à l'entrée de l'étage 18b est transformé, à la sortie de celui-ci en un signal w qui croît dans le temps avec une allure logarithmique.

Dans ce qui précède, les conditions $\alpha kK = 1$ et $\alpha k = K'$ sont réalisées par réglage du coefficient d'atténuation $\alpha$. Il n'est pas exclu, en variante, de se passer du circuit de mise à l'échelle et de régler le gain K ou le coefficient K' en fonction de k, de manière que $kK = 1$ ou que $K' = k$. Dans ce cas, la valeur de k peut être obtenue en mesurant la variation de v pendant un intervalle de temps $\Delta t$ déterminé.

REVENDICATIONS

1.      Procédé de détection de la réponse du coeur à une impulsion électrique de stimulation appliquée à une électrode en contact avec le coeur, par détection dans un signal représentant le potentiel de ladite électrode d'une composante produite par la contraction provoquée du coeur, caractérisé en ce que :

- on recueille, après la fin de l'impulsion de stimulation, un premier signal v qui représente le potentiel d'électrode en circuit ouvert et dont la variation dans le temps t, à partir d'un instant origine $t_o$ est de forme sensiblement logarithmique : $v = v_o + k.\mathrm{Log}\,\dfrac{t}{t_o}$, $v_o$ étant la valeur de v à l'instant $t_o$ et k étant un paramètre lié notamment à la durée et à l'amplitude de l'impulsion de stimulation,

- on convertit le premier signal v en un second signal dont la variation dans le temps est sensiblement linéaire et

- on détecte dans ledit second signal une composante non linéaire représentant le signal produit par la contraction provoquée du coeur.

2.      Procédé selon la revendication 1, caractérisé en ce que la conversion du premier signal comprend une phase de transformation par une fonction f de type exponentiel $f(x) = Ae^{Kx}$, A et K étant deux paramètres.

3.      Procédé selon la revendication 2, caractérisé en ce que ladite conversion comprend aussi une phase d'atténuation du premier signal par un coefficient $\alpha$ tel que $\alpha kK = 1$.

4.      Procédé selon la revendication 2, caractérisé en ce que ladite conversion comprend aussi une phase de réglage du paramètre K de sorte que K = k.

5.      Procédé selon la revendication 1, caractérisé en ce que la conversion du premier signal comprend une phase de génération d'un signal de référence $v_{ref}$ variant de façon logarithmique tel que $v_{ref} = v'_o + K'\,\mathrm{Log}\,\dfrac{t}{t_o}$, $v'_o$ étant la valeur de $v_{ref}$ à l'instant $t_o$ et K' étant un paramètre, et une phase de comparaison du premier signal v avec le signal de référence.

6.      Procédé selon la revendication 5, caractérisé en ce que ladite conversion comprend aussi une phase d'atténuation du premier signal par un coefficient $\alpha'$ tel que $\alpha'k = K'$.

7.      Procédé selon la revendication 6, caractérisé en ce que ladite conversion comprend aussi une phase de réglage du paramètre K' de sorte que K' = k.

8.      Dispositif de détection de la réponse du coeur à une impulsion électrique de stimulation appliquée à une électrode en contact avec le coeur, par détection dans un signal représentant le potentiel de ladite électrode d'une composante produite par la contraction provoquée du coeur, dispositif caractérisé en ce qu'il comporte :

        - un interrupteur (I1) commandé destiné à placer l'électrode (1) en circuit ouvert en réponse à la réception d'un signal de commande (S1), et

        - un circuit de détection comprenant un circuit d'entrée (10, 10') relié à l'électrode (1) pour recueillir un premier signal v qui représente le potentiel d'électrode; un circuit convertisseur (11-14; 11', 17', 18') recevant ledit premier signal et fournissant sur sa sortie un second signal variant dans le temps de façon sensiblement linéaire lorsqu'il reçoit un signal variant dans le temps de façon sensiblement logarithmique; et un détecteur (15, 16; 15') recevant ledit second signal et destiné à détecter dans celui-ci une composante non linéaire représentant le signal produit par la contraction provoquée du coeur.

9.      Dispositif selon la revendication 8, caractérisé en ce que le circuit convertisseur comprend un circuit de normalisation (11, 12) recevant ledit premier signal et délivrant un signal normalisé et un circuit (13) à fonction de transfert exponentielle qui reçoit le signal normalisé et délivre ledit second signal.

10.      Dispositif selon la revendication 9, caractérisé en ce que le détecteur comprend un dérivateur (14) recevant ledit second signal et un circuit de filtrage (15) branché à la sortie du dérivateur pour extraire la composante non continue de la dérivée dudit second signal.

11.      Dispositif selon la revendication 8, caractérisé en ce que le circuit convertisseur comprend un circuit de normalisation (13') recevant ledit premier signal et délivrant un signal normalisé, un générateur (18') de signal de référence dont la variation dans le temps est logarithmique et un soustracteur (17')
recevant le signal normalisé et le signal de référence et délivrant
ledit second signal représentatif de la différence entre les signaux
normalisé et de référence.

12.        Dispositif selon l'une quelconque des revendications 9
à 11, caractérisé en ce que le circuit de normalisation comprend
un circuit atténuateur (12, 12') à gain commandé.

Fig.1

Fig.2

Fig.3

Fig.4

Fig.5

# RAPPORT DE RECHERCHE EUROPEENNE

**0105784**

Numéro de la demande

Office européen
des brevets

EP 83 40 1846

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| A | DE-A-2 800 524 (AMERICAN OPTICAL)<br>* Page 4, alinéa 2; page 12, 2 derniers alinéas; page 13; page 14, alinéas 1,2 * | 1-4 | A 61 N 1/36<br>A 61 N 1/08 |
| A | FR-A-2 431 296 (MEDTRONIC)<br>* Page 19; page 20, ligne 35 - page 21, ligne 14 * | 1 | |
| A | EP-A-0 017 848 (VITATRON)<br>* Page 9, lignes 5-33 * | 1 | |
| | ----- | | |

| DOMAINES TECHNIQUES RECHERCHES (Int. Cl. ³) |
|---|
| A 61 N<br>A 61 B |

Le présent rapport de recherche a été établi pour toutes les revendications

| Lieu de la recherche<br>LA HAYE | Date d'achèvement de la recherche<br>12-12-1983 | Examinateur<br>SIMON J.J.E. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82